(19) Europäisches Patentamt
European Patent Office
Office européen des brevets

(11) **EP 4 785 932 A1**

(12) **EUROPEAN PATENT APPLICATION**

(43) Date of publication:
**05.08.2026 Bulletin 2026/32**

(21) Application number: **25155391.3**

(22) Date of filing: **31.01.2025**

(51) International Patent Classification (IPC):
**A61K 9/20** (2006.01)    **A61K 47/26** (2006.01)

(52) Cooperative Patent Classification (CPC):
**A61K 9/2095; A61K 47/26**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Nederlandse Organisatie voor toegepast-natuurwetenschappelijk onderzoek TNO 2595 DA 's-Gravenhage (NL)**

(72) Inventors:
• **Van Bommel, Kjeld Jacobus Cornelis 2595 DA 's-Gravenhage (NL)**
• **Van Ee, Renz Jeroen 2595 DA 's-Gravenhage (NL)**

(74) Representative: **V.O. P.O. Box 87930 2508 DH Den Haag (NL)**

(54) **EXTRUSION PRINTING FORMULATION**

(57) The invention is aimed at providing a bioactive formulation suitable for printing, preferably 3D printing, the formulation comprises: a first and a second excipient and a bioactive substance that is easily interchangeable with either the first or second excipient. While heating and mixing the formulation a slurry comprising a matrix and a particulate matter is formed such that the formulation may have the required flowability during printing and the dimensional stability to create a final product.

FIG 1

## Description

TECHNICAL FIELD OF THE INVENTION

The invention relates to a bioactive formulation suitable for 3D printing.

BACKGROUND OF THE INVENTION

3D printing of bioactive compositions such as a pharmaceutical product or a nutraceutical product is gaining increased traction due to the unique possibilities like personalized medicine and nutrition, the production of small size batches, increased flexibility, local or on demand production it may offer. Consequently, researchers have investigated various 3D printing technologies to manufacture a bioactive product such as pharmaceutical tablet. An example of 3D printing is extrusion printing, a commonly used Additive Manufacturing process that involves the continuous or noncontinuous flow of material via an, optionally heated, nozzle and the deposition of material at a specified pattern in a layer-by-layer arrangement to build a 3D object. The flow of material may be supplied for instance from a semi-solid material, a thermoplastic material, a slurry, and/or a dispersion as long as sufficient flow can be achieved during extrusion.

When printing a bioactive composition, the formulation, during processing, needs to have a sufficient flow such that the formulation may be extrudable. Typically, sufficient flow may refer to providing a flow to pass through a nozzle or orifice, additionally the formulation must have the required dimensional stability after extrusion such that the extruded material no longer flows after deposition and maintains its 3D-printed shape. Too much material flow may result in a partial loss of the desired shape or may even result in a complete print failure. Typically, when printing a pharmaceutical tablet, the formulation in extrusion printing comprises a meltable matrix and an active pharmaceutical ingredient (API).

Today, for each specific bioactive substance, like for example an API, a new formulation suitable for 3D printing needs to be developed to ensure that the formulation has the required flowability as well as dimensional stability. Therefore, the development of a new printable bioactive formulation is considered time consuming, as multiple formulations need to be developed and further subjected to printing trials before finding a suitable bioactive formulation achieving the right extruding properties as well as the required dimensional stability after deposition.

At present, in formulations suitable for extrusion printing like for example semi-solid extrusion, typically an additional component like for example a solvent may be provided to reduce the viscosity of the formulation improving the extrudability of the formulation. The disadvantage of providing a solvent may be the removal of the solvent immediately after deposition requiring additional process steps, to provide the required dimensional stability.

As such the prior art does not provide any solution or formulation that may reduce the time needed to develop a bioactive 3D printable formulation.

SUMMARY

The present invention is aimed at providing a bioactive formulation suitable for 3D printing such as a pharmaceutical formulation that may be able to reduce the time needed to develop and/or produce a new bioactive formulation. Additionally, the present invention may allow to modify existing formulations and therefore one may not need to start a completely new research program. For example, a bioactive substance such as an API may be easily substituted with an excipient present in the formulation, such that the time required to optimize the printing conditions such as temperature, pressure, or speed may be reduced.

The present invention is directed to bioactive formulation suitable for 3D printing. The formulation comprises: a first excipient and a second excipient and/or a bioactive substance, the first excipient has a lower thermal transition temperature compared to the second excipient such that the bioactive composition forms a slurry during heating and/or mixing above the thermal transition temperature of the first excipient. Optionally, the formulation may comprise more than 2 excipients such as a third or fourth excipient. A slurry may be described as a mixture of small solid particles suspended in a liquid or semiliquid material. The slurry comprises the first excipient in a viscoelastic form acting as a matrix and the second excipient in solid form acting as particulate matter. In the bioactive formulation, a bioactive substance is provided by either substituting at least part of the first excipient when the bioactive substance acts as part of the matrix or at least part of the second excipient when the bioactive substance acts as part of the particulate matter. The particulate matter is capable of providing the slurry in which it is present with the required dimensional stability after 3D printing.

The advantage of such formulation may be that the formulation may be 3D-printed like for example when extruding the formulation, independent of the amount of bioactive substance, reducing the time needed to develop new compositions,

In a further embodiment, the second excipient present in the bioactive formulation may be completely substituted with the bioactive substance.

In yet a further embodiment, the first excipient present in the bioactive formulation may be completely substituted with the bioactive substance.

In yet another embodiment, the matrix may be present in a concentration in the range of 25 - 70 wt.%, preferably in the range of 30 - 65 wt.%, more preferably in the range of 35 - 60 wt.%.

In a further embodiment, the particulate matter may be present in a concentration in the range of 30 - 75 wt.%, preferably in the range of 35 - 70 wt.%, more preferably in the range of 40 - 65 wt.%.

Providing a formulation wherein the first or the second excipient is replaced with a bioactive substance such as an active pharmaceutical ingredient (API) may have the advantage of reducing the development time needed to design a bioactive formulation as interchanging the bioactive substance with either the first or the second excipient may result in a reduction of iterations to design the best formulation. Additionally, as the printing conditions are defined for the formulation comprising the first and second excipient, limited or potentially no experiments may be required to find the optimized 3D printing parameters. Further, as a formulation according to the invention may have the advantage of enabling the design of bioactive formulation independent of the amount of bioactive substance, it may possibly result in a higher amount of bioactive substance present in the formulation compared to conventional production methods, or consequently may reduce the size of an existing bioactive product while maintaining the same amount of bioactive substance in the bioactive product. Further, depending on the choice of the first and second excipient, and the bioactive substance, a too high concentration of a meltable or a softened matrix may reduce the dimensional stability of the 3D printed formulation. A too low concentration of a meltable or softenable matrix may negatively affect the extrudability of the material in the printing process. The concentration ranges may depend on the selection of the components present in the bioactive formulation and generally involves a long trial and error period. The formulation according to the invention may provide the formulation with a universal character such that sufficient dimensional stability may be achieved immediately after deposition such that the use of a solvent may be reduced or even avoided, excluding a drying step after deposition of the extruded material.

In a preferred embodiment, the average diameter of the particulate matter may be defined as at least $1/10^{th}$, preferably $1/20^{th}$ of a die orifice suitable for 3D printing. Typically, the particulate matter may have a particle size (D90) of maximum 100 $\mu$m, preferably maximum 60 $\mu$m, more preferably maximum 50 $\mu$m, most preferably maximum 40 $\mu$m, when using a nozzle diameter of 1 mm, as typically employed for pharma 3D printing.

An average diameter of the particulate matter that is at least $1/10^{th}$ of a die orifice may have the benefit of reducing the probability of blocking the orifice during printing and therefore may reduce downtime of the processing equipment. Mostly a die orifice of 1 mm or smaller may be used to print the bioactive formulation; accordingly the particle size of the particulate matter is ≤ 100 $\mu$m.

In another embodiment, the first excipient may have a thermal transition temperature between 30 and 200°C, preferably between 40 and 100°C, more preferably between 50 and 80°C.

A thermal transition temperature of at least 40°C may have the advantage that the bioactive formulation after printing may not melt at ambient conditions. In certain circumstances it may be more opportune to increase the lower limit of the thermal transition temperature to 50°C especially in cases where the ambient temperature may reach 40°C. The upper limit of 200°C may avoid thermal degradation of the compounds used in the bioactive formulation. In particular, when confronted with sensitive APIs typically the temperature may not increase to close to, or above the degradation temperature of the APIs or any temperature where undesired reactions may occur within the formulation. The degradation temperature of APIs may be regarded as common knowledge for the skilled person.

In an embodiment, the particulate matter may not dissolve in the first excipient.

The particulate matter may not fully dissolve into the first excipient as otherwise no slurry may be formed during heating and/or mixing. Consequently a dissolved particulate matter may not help to increase viscosity and/or may not provide the required dimensional stability after depositing the bioactive formulation. In certain occasions, a limited amount of particulate matter may dissolve into the matrix..

In an embodiment, densities of the particulate matter and matrix may be present in a ratio in the range of 0.66 - 1.5, preferably in the range of 0.9 - 1.1.

A density ratio in the range of 0.66 -1.5 between the particulate matter and matrix may reduce the probability of sedimentation during storage or during processing. Sedimentation may negatively affect the quality of the bioactive formulation after printing as sedimentation may result in concentration fluctuations of the bioactive substance in the formulation and ultimately in the final product.

In yet an embodiment, the first excipient may have a viscosity of at least 2 Pa.s, preferably at least 10 Pa.s. The processing temperature of the formulation may be chosen such that the viscosity reduces to at least 2 Pa.s, preferably to at least 10 Pa.s without negatively affecting the bioactive substance present in the formulation. Therefore, the processing temperature is chosen in function of the formulation.

Similarly, as with the density ratio, the viscosity of the first excipient or matrix may affect the sedimentation or floatation behavior of the particulate matter. A higher matrix viscosity may counterbalance sedimentation as a higher viscosity may increase the force needed for sedimentation or floatation and therefore may reduce the probability of sedimentation.

In another embodiment, no chemical reactions may occur in the formulation between the first excipient, the bioactive substance, or the second excipient.

In another preferred embodiment, the bioactive substance may be an active pharmaceutical ingredient (API).

## BRIEF DESCRIPTION OF THE DRAWINGS

Figure 1 shows a bioactive formulation comprising a bioactive substance, a first excipient and a second excipient.

Figure 2A displays the influence of the particle diameter on sedimentation and/or flotation rate.

Figure 2B displays the influence of the particle density on sedimentation and/or flotation rate.

Figure 2C demonstrates the influence of the matrix density on de sedimentation and/or flotation rate.

Figure 2D demonstrates the influence of the matrix viscosity on de sedimentation and/or flotation rate.

Figure 3 shows a syringe as domain comprising the bioactive formulation according to the invention.

## DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise, it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

3D printing, such as semi solid extrusion of a bioactive formulation is determined by the extrudability of the formulation after heating and/or mixing when producing the formulation and before subjecting the formulation to 3D printing while the dimensional stability of the formulation may be important immediately after depositing the formulation.

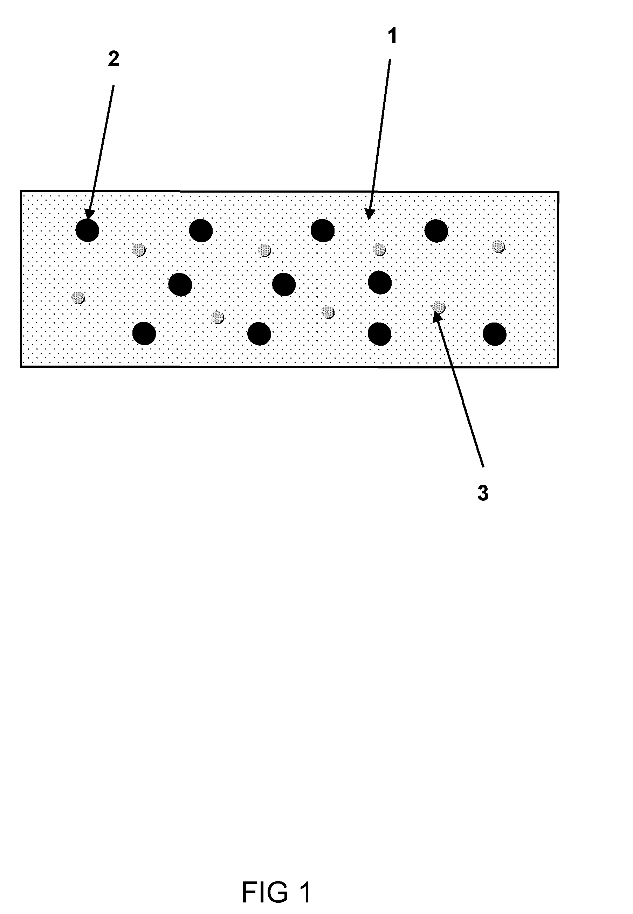

An extrudable bioactive formulation indicates on the one hand that the formulation has a viscosity (under shear) that is low enough to allow extrusion and flow through a nozzle/orifice being employed (the nozzle may have a certain shape, length, opening, diameter). The viscosity of the formulation may be the result of a combination of the matrix and the particulate matter but upon exposure to a temperature above the thermal transition temperature of the matrix, the matrix may start to melt or flow which may reduce the viscosity of the formulation upon 3D printing. Typically, the matrix may, due to its flow characteristics, act as a viscoelastic matrix. On the other hand, dimensional stability requires the formulation to stop flowing after deposition, indicating that the formulation must solidify as soon as possible. Dimensional stability may refer to a property of a bioactive formulation that allows it to maintain its original shape after depositing the formulation. Solidification often involves a phase transition which is associated with substantial amounts of energy and hence may increase the processing time. High processing times may not be compatible with 3D printing as typically 3D printing is confronted with processing times of a few seconds drastically reducing the available time to harden the formulation. Therefore, a second excipient and/or bioactive substance with a higher transition temperature compared to the first excipient is introduced to provide the formulation with the required dimensional stability. Throughout the application viscosity may refer to a measure of a fluid's resistance to flow and hence describing the internal friction of a moving fluid. In the present application the viscosity is measured as a function of the shear rate at the processing/extruding temperature. The viscosity measurements were performed with an Anton-Paar MCR 302 rheometer equipped with a plate-plate geometry (diameter 25 mm) subsequently a representative viscosity value was selected at a shear rate of 100 $s^{-1}$. An example of a bioactive formulation is shown in figure 1. The bioactive formulation depicted in figure 1 comprises a first excipient 1, a second excipient 2 and bioactive substance 3. In figure 1 the formulation is shown after mixing and/or heating above the thermal transition temperature of the first excipient 1. Thermal transition temperature may refer to a temperature at which a material undergoes a notable change in its physical properties due to thermal energy. In the application this typically indicates that the material may start to flow under an applied pressure. A typical example of a thermal transition temperature may be the glass transition temperature, or the melting temperature of a specific material in the formulation like for example the first excipient. Therefore, the first excipient 1 may already be in a molten or softened state such that the first excipient already forms a matrix. The particulate matter in figure 1 is composed of both the second excipient 2 as well as the bioactive substance 3 and accordingly together with the matrix forms a slurry that may be deposited using 3D printing.

Accordingly, the present invention provides a bioactive formulation comprising a first and a second excipient, the first excipient having a lower thermal transition temperature compared to the second excipient such that the bioactive composition forms a slurry during heating and/or mixing above the thermal transition temperature of the first excipient. A slurry may typically comprise solid particles like for example the particulate matter present in the formulation and a molten or softened material such as the matrix of the formulation after subjecting the formulation to a temperature above the thermal transition temperature of the first excipient such that it may be able to flow. The slurry comprises the first excipient in a viscoelastic form acting as a matrix and the second excipient in solid form acting as a particulate matter. The particulate matter preferably does not substantially, preferably does not, flow at the thermal transition temperature of the bioactive formulation. In the bioactive formulation, a bioactive substance is provided by either exchanging at least part of the first excipient when the bioactive substance acts as part of the matrix or at least part of the second excipient when the bioactive substance acts as part of the particulate matter. The particulate matter is capable of providing the required dimensional stability after 3D printing. Respectively, the first excipient acting as a matrix provides for the required flow when heating and/or mixing and during printing while the particulate matter provides for dimensional stability. Typically, the dimensional stability is defined as the percentage difference between the final dimension minus the initial dimension of the 3D printed produced divided by the initial dimension multiplied by 100. Appropriate dimensional stability refers to a percentage difference smaller than 10%; preferably smaller than 5%, more preferably smaller than 2%, most preferably smaller than 1%.

Typical examples of a first or second excipient like for example bioactive acceptable polymers may be selected from, but not limited to, water-soluble polymers, water-dispersible polymers or water-swellable polymers or any mixture thereof. Polymers may be considered water-soluble if they form a clear homogeneous solution in water. Other examples may include hard fats such as glycerides like mono-, di, or triglycerides

Preferred excipients may be selected from the group comprising:

- homopolymers and copolymers of N-vinyl lactams, especially homopolymers and copolymers of N-vinyl pyrrolidone, e.g., polyvinylpyrrolidone (PVP), copolymers of N-vinyl pyrrolidone and vinyl acetate or vinyl propionate.

- cellulose esters and cellulose ethers, in particular methylcellulose and ethyl-cellulose, hydroxyalkylcellulose, in particular hydroxypropyl cellulose, hydroxyalkylalkylcelluloses, in particular hydroxypropyl methylcellulose, cellulose phthalates, or succinates, in particular cellulose acetate phthalate and hydroxypropyl methylcellulose phthalate, hydroxypropyl methylcellulose succinate or hydroxypropyl methylcellulose acetate succinate.

- high molecular poly-alkylene oxides such as polyethylene oxide and polypropylene oxide and copolymers of ethylene oxide and propylene oxide,

- polyvinyl alcohol-polyethylene glycol-graft copolymers (available as Kollicoat® IR from BASF AG, Ludwigshafen, Germany),

- polyacrylates and polymethacrylates such as methacrylic acid/ethyl acrylate copolymers, methacrylic acid/methyl methacrylate copolymers, butyl methacrylate/2-dimethylaminoethyl methacrylate copolymers, poly (hydroxyalkyl acrylates), poly (hydroxyalkyl methacrylate's),

- polyacrylamides,

- vinyl acetate polymers such as copolymers of vinyl acetate and crotonic acid, partially hydrolyzed polyvinyl acetate (also referred to as partially saponified "polyvinyl alcohol"),

- polyvinyl alcohol,

- oligo- and polysaccharides such as carrageenan's, galactomannans and xanthan gum, or any combination thereof.

Typical examples of bioactive substances such as a pharmaceutical or nutraceutical substance may comprise, but not limited to:

- analgesic and anti-inflammatory drugs such as fentanyl, indomethacin, ibuprofen, naproxene, diclofenac, diclofenac sodium, fenoprofen, acetylsalicylic acid, ketoprofen, nabumetone, paracetamol, piroxicam, meloxicam, tramadol, and COX-2 inhibitors such as celecoxib and rofecoxib.

- anti-arrhythmic drugs such as procainamide, quinidine and verapamil;

- antibacterial and antiprotozoal agents such as amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin sodium, halofantrine, isoniazid, kanamycin sulphate, lincomycin, mefloquine, minocycline, nafcillin sodium, nalidixic acid, neomycin, nortloxacin, ofloxacin, oxacillin, phenoxymethyl-penicillin potassium, pyrimethamine-sulfadoxime and streptomycin;

- anti-coagulants such as warfarin;

- antidepressants such as amitriptyline, amoxapine, butriptyline, clomipramine, desipramine, dothiepin, doxepin, fluoxetine, reboxetine, amineptine, selegiline, gepirone, imipramine, lithium carbonate, mianserin, milnacipran, nortriptyline, paroxetine, sertraline and 3-[2-[3,4-dihydrobenzofuro[3,2-c]pyridin-2(1H)-yl]ethyl]-2-methyl-4H-pyrido[1,2-a]pyrimidin-4-one;

- anti-diabetic drugs such as glibenclamide and metformin;

- anti-epileptic drugs such as carbamazepine, clonazepam, ethosuximide, gabapentin, lamotrigine, levetiracetam, phenobarbitone, phenytoin, primidone, tiagabine, topiramate, valpromide and vigabatrin;

- antifungal agents such as amphotericin, clotrimazole, econazole, fluconazole, flucytosine, griseofulvin, itraconazole, ketoconazole, miconazole nitrate, nystatin, terbinafine and voriconazole;

- antihistamines such as astemizole, cinnarizine, cyproheptadine, decarboethoxyloratadine, fexofenadine, flunarizine, levocabastine, loratadine, norastemizole, oxatomide, promethazine and terfenadine;

- anti-hypertensive drugs such as captopril, enalapril, ketanserin, lisinopril, minoxidil, prazosin, ramipril, reserpine, terazosin and telmisartan;

- anti-muscarinic agents such as atropine sulphate and hyoscine;

- antineoplastic agents and antimetabolites such as platinum compounds, such as cisplatin and carboplatin; taxanes such as paclitaxel and docetaxel; tecans such as camptothecin, irinotecan and topotecan; vinca alkaloids such as vinblastine, vindecine, vincristine and vinorelbine; nucleoside derivatives and folic acid antagonists such as 5-fluorouracil, capecitabine, gemcitabine, mercaptopurine, thioguanine, cladribine and methotrexate; alkylating agents such as the nitrogen mustards, e.g. cyclophosphamide, chlorambucil, chiormethine, iphosphamide, melphalan, or the nitrosoureas, e.g. carmustine, lomustine, or other alkylating agents, e.g. busulphan, dacarbazine, procarbazine, thiotepa; antibiotics such as daunorubicin, doxorubicin, idarubicin, epirubicin, bleomycin, dactinomycin and mitomycin; HER 2 antibodies such as trastuzumab; podophyllotoxin derivatives such as etoposide and teniposide; famesyl transferase inhibitors; anthrachinon derivatives such as mitoxantron;

- anti-migraine drugs such as alniditan, naratriptan and sumatriptan.

- anti-Parkinsonian drugs such as bromocryptine mesylate, levodopa and selegiline.

- antipsychotic, hypnotic and sedating agents such as alprazolam, buspirone, chlordiazepoxide, chlorpromazine, clozapine, diazepam, flupenthixol, fluphenazine, flurazepam, 9-hydroxyrisperidone, lorazepam, mazapertine, olanzapine, oxazepam, pimozide, pipamperone, piracetam, promazine, risperidone, selfotel, seroquel, sertindole, sulpiride, temazepam, thiothixene, triazolam, trifluperidol, ziprasidone and zolpidem.

- anti-stroke agents such as lubeluzole, lubeluzole oxide, riluzole, aptiganel, eliprodil and remacemide.

- antitussives such as dextromethorphan and laevodropropizine.

- antivirals such as acyclovir, ganciclovir, loviride, tivirapine, zidovudine, lamivudine, zidovudine/lamivudine, didanosine, zalcitabine, stavudine, abacavir, lopinavir, amprenavir, nevirapine, efavirenz, delavirdine, indinavir, nelfinavir, ritonavir, saquinavir, adefovir and hydroxyurea;

- beta-adrenoceptor blocking agents such as atenolol, carvedilol, metoprolol, nebivolol and propanolol;

- cardiac inotropic agents such as amrinone, digitoxin, digoxin and milrinone;

- corticosteroids such as beclomethasone dipropionate, betamethasone, budesonide, dexamethasone, hydrocortisone, methylprednisolone, prednisolone, prednisone and triamcinolone.

- disinfectants such as chlorhexidine;

- diuretics such as acetazolamide, furosemide, hydrochlorothiazide and isosorbide;

- enzymes;

- essential oils such as anethole, anise oil, caraway, cardamom, cassia oil, cineole, cinnamon oil, clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol and thyme.

- gastro-intestinal agents such as cimetidine, cisapride, clebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, prucalopride, rabeprazole, ranitidine, ridogrel and sulphasalazine

- haemostatics such as aminocaproic acid;

- lipid regulating agents such as atorvastatin, fenofibrate, fenofibric acid, lovastatin, pravastatin, probucol and simvastatin;

- local anesthetics such as benzocaine and lignocaine;

- opioid analgesics such as buprenorphine, codeine, dextromoramide, dihydrocodeine, hydrocodone, oxycodone and morphine;

- parasympathomimetics and anti-dementia drugs such as AIT-082, eptastigmine, galanthamine, metrifonate, milameline, neostigmine, physostigmine, tacrine, donepezil, rivastigmine, sabcomeline, talsaclidine, xanomeline, memantine and lazabemide.

- peptides and proteins such as antibodies, becaplermin, cyclosporine, tacrolimus, erythropoietin, immunoglobulins and insulin.

- sex hormones such as estrogens: conjugated estrogens, ethinyloestradiol, mestranol, estradiol, oestriol, oestrone; progestogens; chlormadinone acetate, cyproterone acetate, 17-deacetyl norgestimate, desogestrel, dienogest, dydrogesterone, ethynediol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimate, norgestrel, norgestrienone, progesterone and quingestanol acetate;

- stimulating agents such as sildenafil, vardenafil.

- vasodilators such as amlodipine, buflomedil, amyl nitrite, diltiazem, dipyridamole, glyceryl trinitrate, isosorbide dinitrate, lidoflazine, molsidomine, nicardipine, nifedipine, oxpentifylline and pentaerythritol tetranitrate.

- their N-oxides, their pharmaceutically acceptable acid or base addition salts and their stereochemically isomeric forms.

- Examples for nutraceuticals may be resveratrol as an antioxidant, soluble dietary fiber products, broccoli (sulphane) as a preservative for cancer, and soy or clover (isoflavonoids) for improving arterial health.

- Other nutraceuticals examples may be flavonoids, antioxidants, alpha-linoleic acid from flax seed, beta-carotene from marigold petals or antocyanins from berries. Sometimes the expression neutraceuticals is used as synonym for nutraceuticals.

- Other examples of nutraceuticals may include Carotenoids such as lycopene, dietary enzymes such as papain and bromelain, hydrolyzed proteins, mineral supplements, phytonutrients, prebiotic and probiotic supplements, dietary fiber supplements, vitamin supplements, herbal products such as: echinacea, ginger, garlic, ginseng, onion, licorice root, turmeric.

- Further examples of nutraceuticals may include 5 HTP, acai, aloe, astragalus, ayurvedic herbs, B vitamins, bee products, bilberry, bitter melon, black cohosh root, calcium, cascara sagrada, cat's claw, cayenne, chamomile, chromium, collagen, combination herbs, CoQ10, cranberry, DHEA, digestive enzymes, drinks, elderberry, evening primrose, fenugreek, fish/animal oils, fruit and vegetable supplements, garcinia, gelatin, ginkgo biloba, glucosamine/chondroitin, goji juice, goldenseal, grape seed extract, grapefruit seed extract, green foods, green tea, gymnema sylvestre, hawthorne, hemp CBD, homeopathics, horny goat weed, iron, kava kava, kombucha, lycopene, maca, magnesium, mangosteen juice, meal supplements, melatonin, milk thistle, MSM, mullein, multivitamins, mushrooms, noni juice, olive leaf extract, oregano, other Chinese herbs, other minerals, others specialty, papaya, Pau D'Arco, peppermint and other mints, pills, pine bark extract, plant oils, potassium, synbiotics, psyllium, red yeast rice, rosehips, rosemary, SAMe, saw palmetto, selenium, senna, slippery elm, spirulina, sports nutrition powders, St. John's Wort, stevia, tribulus terrestris, valerian, vitamin A, vitamin C, vitamin D, vitamin E, vitamin K, H, combo vitamins, other vitamins, vitex, willowbark, zinc.

Typically, the particulate matter comprises the second excipient and/or the bioactive substance, preferably the bioactive substance is an active pharmaceutical ingredient. Preferably, the particulate matter may not form large aggregates in the slurry as aggregate formation may result in large particles that may block the nozzle or may result in a scenario wherein a limited number of particles, when printing a tablet, may result in an undesirable API content such that either too much or too little API would be present in the formulation. Further, aggregates may comprise undesired entrapped air bubbles as during aggregate formation air bubbles may be formed when the particulate matter starts to sediment or float upon compacting of multiple aggregates or particulate matter and accordingly enclose air during the formation of larger aggregates.. Typically, the particle size of the particulate matter, the viscosity of the matrix and the difference in density between the particulate matter and the matrix may affect the sedimentation rate of the formulation according to the invention.

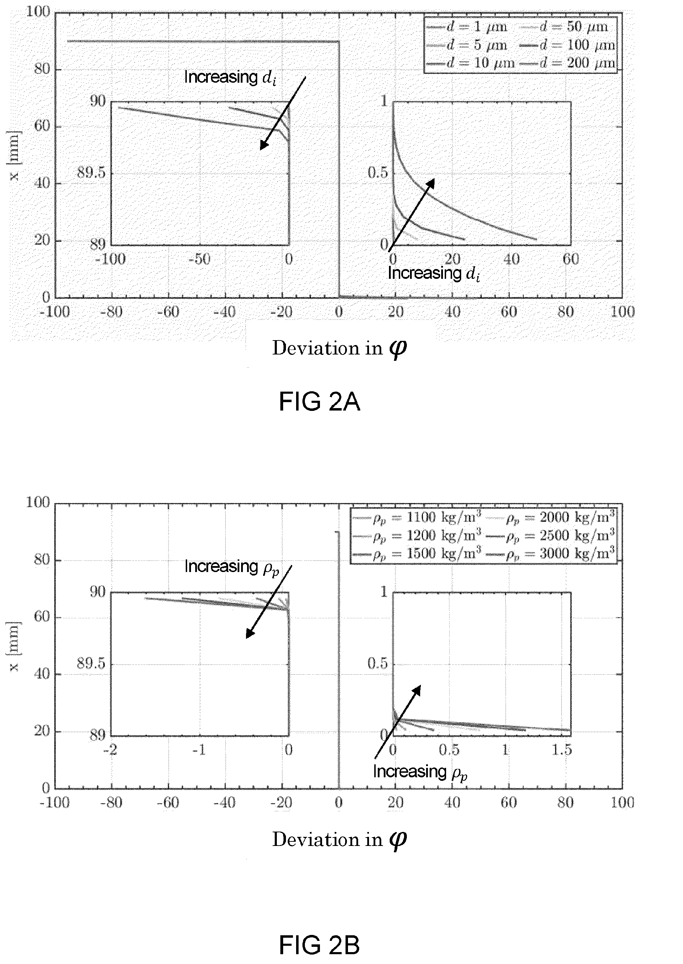

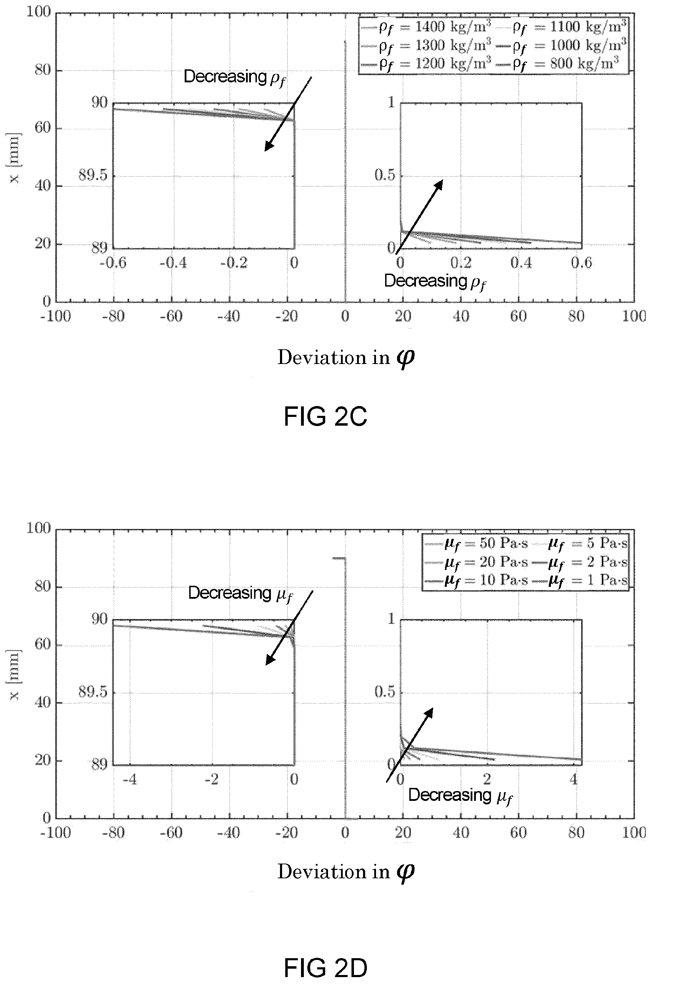

To retrieve more insight into the stability of the formulation, simulations were performed, wherein parameters $d_i$, $\rho_p$, $\rho_f$ and $\mu_f$ were varied, such that the sedimentation/floatation behavior may be investigated when applying static conditions on a single particle phase. The parameters $d_i$, $\rho_p$, $\rho_f$ and $\mu_f$ refer respectively to the particle diameter $d_i$ of the particulate matter like for example the diameter of the second excipient and/or the bioactive substance, the density of the particulate matter $\rho_p$, the density $\rho_f$ and the viscosity $\mu_f$ of the formulation. When developing and/or manufacturing bioactive formulation, sedimentation and/or floatation may be carefully need to be monitored as sedimentation/floatation may affect the API concentration in a tablet manufactured by 3D printing. The simulations were performed with parameters typically applied for a bioactive formulation such as a pharmaceutical tablet. These typical parameters can be found in table 1. The results obtained from the simulations may be found in figures 2A, 2B, 2C and 2D. Figures 2A - 2D express the variation in the volume fraction of the particulate matter $\phi$ starting from $\phi_0$ in function of the position x of a domain like for example the length of a syringe used for depositing the bioactive formulation. Throughout the application x = 0 at the bottom of the domain while x = L is considered is the top of the domain, unless otherwise specified. The sedimentation behavior was examined over a period of 30 min, which may be considered common during printing of a bioactive formulation.

*Table 1*

| $\rho_p$ | 1520 kg/m³ | $\rho_f$ | 1060 kg/m³ |
|---|---|---|---|
| $d_i$ | 10 μm | $\mu_f$ | 11.7 Pa.s |
| $\varphi_{max}$ | 0.63 | T | 53°C |
| $\varphi_0$ | 0.354 | L | 90 mm |

The simulations provided multiple insights into the sedimentation or floatation behavior of a bioactive formulation according to the invention. Firstly, static sedimentation simulations showed that over time the volume particle fraction decreased at a the top of the domain while an increase of the volume fraction was observed at the bottom of the domain, the decrease as well as increase in volume fraction is shown in figures 2A - 2D by respectively a negative variation of $\varphi$ at the top and a positive variation of $\varphi$ at the bottom. The variation at the bottom and top of the domain may be explained by the higher density of the particulate matter compared to the density of matrix, resulting in a downward sedimentation force and subsequently in a descend of the particulate matter.

Figures 2A - 2D further show that the sedimentation or floatation may be affected by varying the parameters $d_i$, $\rho_p$, $\rho_f$ or $\mu_f$. Typically, sedimentation or floatation increases when the particle size or density of the particulate matter increases or when the density or viscosity of the matrix decreases. The relation between sedimentation or floatation on the parameters $d_i$, $\rho_p$, $\rho_f$ or $\mu_f$ can be expressed by the formula:

$$u_i(\varphi_1, ..., \varphi_n) = \frac{d_i^2 * \left(\rho_{p,i} - \rho_f(\varphi_1,...,\varphi_n)\right) * g}{18 * \mu_f(\varphi_1,...,\varphi_n)}$$

Wherein $u_i(\varphi_1, ..., \varphi_n)$ represents the sedimentation speed of for example the second excipient or API present in the particulate matter, $d_i^2$, $\rho_{p,i}$ represent respectively the particle size and density of the second excipient or API present in the particulate matter, $\rho_f(\varphi_1, ..., \varphi_n)$ represents the density of the formulation and may be affected by both the density of the matrix ($\rho_m$) as well as the particulate matter, $\mu_f(\varphi_1, ..., \varphi_n)$ represents the viscosity of the formulation, typically the viscosity of the formulation is linearly depending on the matrix viscosity ($\mu_m$), $\varphi_1$ represent the volume fraction of second excipient or API in the formulation and n represents the amount of compounds present in the particulate matter. The dependency of $\rho_f(\varphi_1, ..., \varphi_n)$ and $\mu_f(\varphi_1, ..., \varphi_n)$ may be described as:

$$\rho_f(\varphi_1, ..., \varphi_n) = \sum_{i=1}^{n} \varphi_i * \rho_{p,i} + \left(1 - \sum_{i=1}^{n} \varphi_i\right) * \rho_m$$

$$\mu_f(\varphi_1, ..., \varphi_n) = \mu_m * (1 - \frac{\varphi_{tot}(\varphi_1, ..., \varphi_n)}{\varphi_{max}})^{-2}$$

Wherein $\varphi_{tot}(\varphi_1, ..., \varphi_n)$ represents the total volume fraction of the particulate matter present in the bioactive formulation and $\varphi_{max}$ represents the maximum volume fraction that may achieved in the formulation, typically this value may be set at 0.63 or 0.75.

The above described simulations may be further understood by referring to Figures 2A - 2D. For a proper understanding, please carefully consider that one parameter from $d_i$, $\rho_p$, $\rho_f$ or $\mu_f$ may vary while to other 3 may be kept constant. When a parameter is constant, the value is indicated as in Table 1 of the application. All graphs display a percentage deviation of the volume fraction in function of the length of a domain like for example a rectangle comprising a length of 90 mm. The percentage deviation may be defined as a difference between $\varphi_0$ (defined as the initial volume fraction after mixing and/or heating the bioactive formulation) and the volume fraction after a certain time for instance 30 minutes divided by $\varphi_0$. A positive deviation may indicate an increase in particulate matter concentration while a negative deviation may refer to a decrease in particulate matter concentration compared to $\varphi_0$.

Figure 2A displays the influence of the particle size of the particulate matter on sedimentation behavior. Particle size (D90) may be described as the diameter at which 90% of the particles in a sample are smaller, and 10% are larger. Figure 2A shows how a variation in particle size may affect sedimentation over a timespan 30 minutes. As aforementioned, it may be noted that the rate of sedimentation increases when the particle size increases, this may typically be observed in the enlarged graphs on the left and right side of figure 2A for respectively the top and bottom of the domains. While simulating the effect of particle size all other parameters were kept constant and indicated in table 1.

Accordingly, a first method to minimize sedimentation may be by changing the particles size of the particulate matter. Typically, settling velocity reduces when the particle size decreases. A smaller particle size may reduce the gravitational forces and therefore may minimize sedimentation. Typically, when considering the simulations and the parameters, the particle size may preferably be below 100 $\mu$m, preferably below 60 $\mu$m, more preferably below 50 $\mu$m, most preferably below 40 $\mu$m. The addition of particles to a material of a certain viscosity may increase the overall viscosity of the bioactive formulation resulting in a reduced flow. The first excipient and optionally the second excipient may be materials that have a temperature dependent viscosity, as the printing may happen at an elevated temperature the viscosity decreases during mixing and/or extruding. Concurrently, after extrusion and exposure to ambient conditions, the first excipient may solidify increasing the viscosity to the point that flow will no longer take place. The particulate matter is introduced to provide the required viscosity such that the flow after deposition may be limited such that the matrix material may have the possibility to increase in viscosity due to the drop in temperature, at which point the overall formulation will become a solid material.

Figure 2B displays the effect of the density of the particulate matter on the sedimentation rate after 30 minutes, for a more severe effect the time may be increased to 60 minutes, 1 day or even 1 week. As shown in figure 2B, sedimentation rate may increase when the density of the particulate matter increases, the increase in sedimentation may typically be observed at the top and bottom of the domain as is shown in the enlarged graphs on the left on right side of Figure 2B for respectively the top and the bottom of the domain. While simulating the effect of particulate matter density all other parameters were kept constant and indicated in table 1.

Figure 2C, on the other hand depicts the influence of the matrix density. As mentioned above, sedimentation may decrease when the matrix density decreases. The difference in sedimentation is typically more pronounced at the top and bottom of the domain as is highlighted in the enlarged graphs in the left and right side of Figure 2C for respectively the top and the bottom of the domain. While simulating the effect of matrix density all other parameters were kept constant and indicated in table 1.

Accordingly, a second method of minimizing sedimentation may be by ensuring that the density ratio, at printing temperature, between the particulate matter and the matrix preferably is in between 0.66 and 1.5, more preferably between 0.9 and 1.1. Simulations have shown that when the density ratio of particulate matter and matrix is between 0.66 and 1.5 that sedimentation may be reduced to a minimum such that a proper product quality may be guaranteed after depositing the bioactive formulation. Simulations demonstrated that the settling velocity reduces when the density of the particulate matter matches the density of the matrix. Figure 2D shows the influence of the matrix viscosity on the rate of sedimentation. Typically, sedimentation may reduce when the matrix viscosity increases, this may again be more noticeable on the top and bottom of the domain as is depicted in the small graphs on the left on right side of Figure 2D. While simulating the effect of matrix viscosity all other parameters were kept constant and indicated in table 1.

Accordingly, a third method of minimizing sedimentation may be by increasing the viscosity of the matrix (of the first excipient at processing temperature) to at least 2 Pa.s, preferably at least 10 Pa.s at the printing temperature. Simulations, using specific conditions as specified in Table 1, have shown that an increase in viscosity may reduce the settling viscosity and therefore reduce the probability of sedimentation. The formula and the results of the simulations may indicate that the particle size of the particulate matter may have the largest impact on the sedimentation behavior of the bioactive formulation. Further, even though the impact of parameters $\rho_p$, $\rho_m$ or $\mu_m$ is more pronounced when time increases, they may nevertheless influence the sedimentation behavior and therefore these parameters may be tuned in order to obtain a more stable bioactive formulation. Accordingly, to prevent sedimentation, typically the particulate matter used in bioactive formulations may be ground such that the particle size $d_i$ decreases. Additionally, it may optionally be appropriate to choose a density of the matrix that may be close to the density of the particulate matter and/or to increase to viscosity of the matrix balancing the flowability of the formulation during deposition. (while still making sure that adequate flowability of the formulation is ensured for processing).

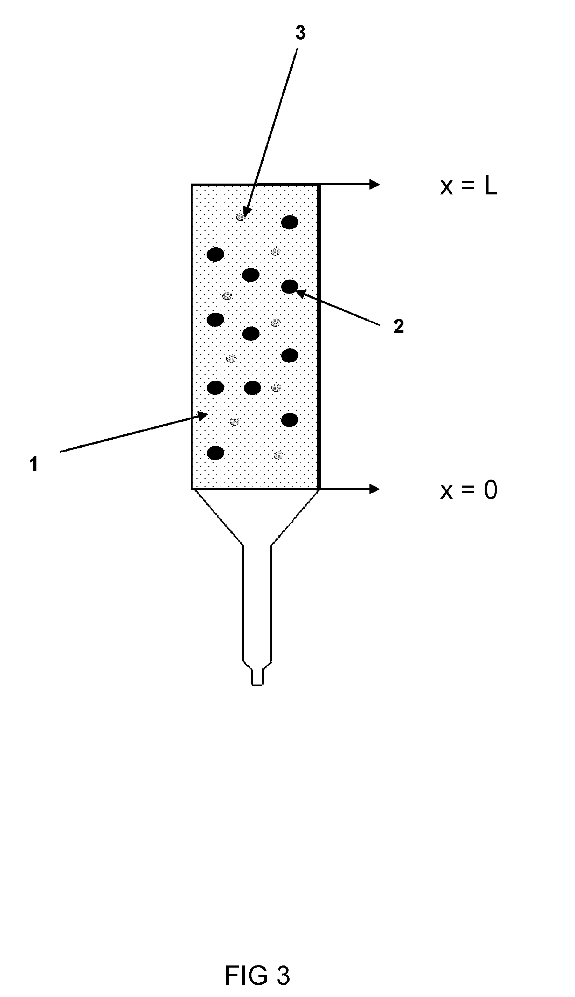

The insights obtained during the simulations were subsequently extrapolated to bioactive formulations according to the invention. Additional simulations were performed on 3 different bioactive formulations wherein the first excipient was Kolliphor P407 while the particulate matter was varied between sorbolac 400 (formulation 1), caffeine (formulation 2) or a combination of an active pharmaceutical ingredient and sorbolac 400 (formulation 3). For the simulations, a syringe was used as domain as is shown in figure 3. Figure 3 further illustrates which area may be used to simulate the sedimentation rate in a syringe, to evaluate the sedimentation, the top of the syringe may be designated as $x = L$, while the bottom location, denoted as $x = 0$, may be found were the width of the syringe starts to decreasing. The processing temperature may be selected at 53°C or 80°C and were used to evaluate to extend of sedimentation. Both processing temperatures resulted in respectively a matrix viscosity of 11.7 Pa.s and 2.9 Pa.s. Other parameters such as densities of the different compounds used in the bioactive formulation may be found in Table 1. While abovementioned simulations were performed with a particulate matter typically comprising one particle size for the particulate matter, actual particulate matter used in bioactive formulations often comprises a particle size distribution which may affect the sedimentation behavior. Nevertheless, the simulations revealed that similar insights may be obtained when bioactive formulations, comprising a particulate matter with a particle size distribution, were simulated. As such, it may be concluded that the particle size of the particulate matter, the densities of the first excipient, second excipient and bioactive substance, as well as the viscosity of the first excipient at the processing temperature may impact sedimentation behavior of the formulation. Further, an increase in processing temperature may facilitate sedimentation as an increase in temperature generally decreases the viscosity of the matrix and consequently may increase sedimentation. Furthermore, when using multiple compounds such as a second excipient and an API as particulate matter, generally these compounds don't have one particle size but may have a particle size distribution. Simulations have shown that similar conclusions as observed in the abovementioned simulations comprising a particulate matter that includes one particle size and one compound, may be drawn when a particulate matter comprises at least two different compounds such as a second excipient and an API. Additionally, the compounds present as particulate matter may have different densities, therefore it may be of interest to decrease the particle size of the compound having the density that is furthest apart from the density of the matrix such that sedimentation or floatation rate may decrease.

Accordingly, by selecting the proper matrix viscosity, the particle size of the particulate matter and/or the density difference between the matrix and particulate matter, sedimentation or floatation may be reduced to a minimum or may even excluded.

The bioactive formulation according to the invention may be produced according to any conventional techniques known be a person skilled in the art. Typical examples may be:

- Placing powders in speed mixing cup and speed mixing to melt the matrix material, whilst mixing, resulting in an air (bubble) free homogeneously mixed dispersion of the particulate matter (API/excipient) in the matrix material. The material may then be transferred (by means of a speed press) into a syringe, which may be placed in the printer. Note: other configurations may be possible.

- Mixing the powders by means of a tumbler or other setup and then pressing the well mixed powders into a billet by means of a high pressure setup. The billet may be placed in a syringe or other holder for use in the printer.

- Mixing the powders by means of a tumbler or other setup and then transferring them into a progression/-melting system (screw extruder or other), which melts, blends and de-airs the mixture to arrive at a molten or softened desired dispersion that may be extruded/printed into a tablet shape.

- Other mixing technologies techniques like for example convective mixing which may be suitable for cohesive or sticky powders, or sheer mixing suitable in achieving uniform particle distribution, fluidized bed mixing may ensure mixing of finer particles, a v type blender may be efficient for mixing free-flowing powders and granules, or ribbon blending which may provide thorough mixing of powders and pastes and of most commonly used nutraceuticals and food products

Further, bioactive formulations were printed using semi-solid extrusion as 3D printing technique. The examples described below the formulations comprised either polyethylene glycol or a polyethylene glycol polypropylene glycol block co-polymer as a first excipient acting as matrix when subjected to a temperature above the thermal transition temperature of the first excipient and either lactose or a combination of an API and a second excipient acting as a particulate matter. The formulations were manufactured by weighing the compound powders in speed mixing cup, premixing by running the speed mixer at 750 rpm for 30 sec, followed by speed mixing twice for 2 min at 3500 rpm such that a homogeneous paste is achieved. Subsequently, the homogeneous paste is transferred by a speed press to a 10 mL syringe with 1 mm nozzle, followed by loading the syringe into an extrusion printer. After the syringe is loaded into the extrusion printer, the syringe together with the nozzle is heated to a temperature of 53°C. Once the homogeneous paste reaches a temperature of 53°C, the paste is printed, on a heated print bed in a tablet design comprising 7-8 layers of a thickness in between 0.3 to 0.4 mm, depending on the print design. Typically, the heated print bed may have a temperature in between 25 and 35 °C depending on the experiment. Optionally, a cold air shower is used to speed up solidification to allow for faster printing. The first excipient used in the experiments has a thermal transition temperature between 42 and 55 °C, being lower than the thermal transition temperature of lactose or the mixture of particulate matter. Consequently, during and after heating and speed mixing, a slurry was formed comprising the softened first excipient as matrix and the lactose or a mixture of the second excipient and API as particulate matter. The experiments demonstrated that successful formulations were designed with a concentration of polyethylene glycol or a polyethylene glycol polypropylene glycol block co-polymer between 35 wt.% and 60 wt.% and a concentration of the particulate matter between 40 wt.% and 65 wt.%.

For the purpose of clarity and a concise description, features are described herein as part of the same or separate embodiments, however, it will be appreciated that the scope of the invention may include embodiments having combinations of all or some of the features described. For example, while embodiments were shown for bioactive formulations, also alternative ways may be envisaged by those skilled in the art having the benefit of the present disclosure for achieving a similar function and result. The various elements of the embodiments as discussed and shown offer certain advantages, such as reducing the time needed to develop and/or produce a new bioactive formulation. Of course, it is to be appreciated that any one of the above embodiments or processes may be combined with one or more other embodiments or processes to provide even further improvements in finding and matching designs and advantages. It is appreciated that this disclosure offers particular advantages to the 3D printing of bioactive formulation, and in general can be applied for any application wherein slurries may need to be printed.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A bioactive formulation suitable for 3D printing, the formulation comprising:

   a. a first excipient;
   b. a second excipient; and
   c. a bioactive substance;
   the first excipient having a lower thermal transition temperature compared to the second excipient such that the bioactive formulation forms a slurry during heating and/or mixing above the thermal transition temperature of the first excipient, the slurry comprising the first excipient in a viscoelastic form acting as a matrix and the second excipient in solid form acting as particulate matter;
   wherein the bioactive substance either acts as part of the matrix or acts as part of the particulate matter such that the bioactive substance substitutes at least a part of either the first excipient or the second excipient;
   and wherein the particulate matter provides for dimensional stability after 3D printing.

2. The bioactive formulation according to claim 1, wherein the average diameter of the particulate matter is defined is at least 1/10th of a print head orifice suitable for 3D printing.

3. The bioactive formulation according to claim 1 or claim 2, wherein the thermal transition temperature is between 40 and 200°C.

4. The bioactive formulation according to any of claims 1-3, wherein the first excipient is present in a concentration in the range of 30 - 56 wt.%.

5. The bioactive formulation according to any of claims 1-4, wherein the particulate matter is present in a concentration in the range of 44 - 70 wt.%.

6. The bioactive formulation according to any of claims 1-5, wherein the particulate matter has a particle size (D90) of maximum 60 $\mu$m.

7. The bioactive formulation according to any of claims 1-6, wherein the particulate matter does not dissolve in the first excipient.

8. The bioactive formulation according to any of claims 1-7, wherein a ratio of the densities of the particular matter and matrix is in the range of 0.66 - 1.5.

9. The bioactive formulation according to any of claims 1-8, wherein the viscosity at processing temperature of the first excipient is at least 2 Pa.s.

10. The bioactive formulation according to any of claims 1-9, wherein no chemical reactions occur in the formulation between the first excipient, the bioactive substance, or the second excipient.

11. The bioactive formulation according to any of claims 1 - 10, wherein the bioactive substance is an active pharmaceutical ingredient (API).

FIG 1

FIG 2A

FIG 2B

FIG 2C

FIG 2D

FIG 3

Europäisches
Patentamt

European
Patent Office

Office européen
des brevets

## EUROPEAN SEARCH REPORT

**Application Number**

EP 25 15 5391

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 417 195 A1 (UNIV HAMBURG EPPENDORF UKE [DE]) 21 August 2024 (2024-08-21) * example 2 * * claims * ----- | 1-11 | INV. A61K9/20 A61K47/26 |
| X | US 2022/071911 A1 (VAN DEN HEUVEL KORINDE [NL]) 10 March 2022 (2022-03-10) * paragraph [0042] * * paragraph [0043] * ----- | 1-11 | |

**TECHNICAL FIELDS SEARCHED (IPC)**

A61K

The present search report has been drawn up for all claims

| Place of search | Date of completion of the search | Examiner |
|---|---|---|
| Munich | 16 July 2025 | Schüle, Stefanie |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
..........................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 15 5391

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2025

| Patent document cited in search report | | Publication date | Patent family member(s) | | Publication date |
|---|---|---|---|---|---|
| EP 4417195 | A1 | 21-08-2024 | EP | 4417195 A1 | 21-08-2024 |
| | | | WO | 2024170473 A1 | 22-08-2024 |
| US 2022071911 | A1 | 10-03-2022 | AU | 2020277668 A1 | 25-11-2021 |
| | | | BR | 112021023189 A2 | 04-01-2022 |
| | | | CN | 113891706 A | 04-01-2022 |
| | | | EP | 3972567 A1 | 30-03-2022 |
| | | | JP | 2022533213 A | 21-07-2022 |
| | | | US | 2022071911 A1 | 10-03-2022 |
| | | | WO | 2020234335 A1 | 26-11-2020 |

EPO FORM P0459

For more details about this annex : see Official Journal of the European Patent Office, No. 12/82